# EUROPEAN PATENT SPECIFICATION

(11) **EP 4 276 652 B1**
(45) Date of publication and mention of the grant of the patent: **18.11.2026**
(21) Application number: 23201094.2
(22) Date of filing: 23.07.2010
(51) Int. Cl.: G06F 16/28, A61B 5/145, A61B 5/00, G16H 20/30, A61M 5/172, G16H 40/60, G16H 50/30, G16H 50/20, G16H 50/50, A61M 5/142

(54) **REAL TIME MANAGEMENT OF DATA RELATING TO PHYSIOLOGICAL CONTROL OF GLUCOSE LEVELS**
ECHTZEITVERWALTUNG VON DATEN IN ZUSAMMENHANG MIT DER PHYSIOLOGISCHEN STEUERUNG VON GLUCOSESPIEGELN
GESTION EN TEMPS RÉEL DE DONNÉES RELATIVES À LA RÉGULATION PHYSIOLOGIQUE DES TAUX DE GLUCOSE

(30) Priority: 23.07.2009 US 22810109 P
(43) Date of publication of application: 15.11.2023
(60) Divisional application: 24180783.3 / 4 404 212
(62) Document number(s) of the earlier application(s) in accordance with Art. 76 EPC: 21190964.3 / 3 936 032; 16187292.4 / 3 173 014; 10739770.5 / 2 456 351
(73) Proprietor: Abbott Diabetes Care Inc., Alameda, CA 94502 (US)
(72) Inventor: WEI, Charles, Fremont, 94555 (US); HAYTER, Gary Alan, Oakland, 94618 (US)
(74) Representative: Boult Wade Tennant LLP

(56) References cited:
- WO-A1-2008/151452
- WO-A1-2009/049252
- US-A1- 2008 300 572
- US-B1- 6 923 763

## Description

### RELATED APPLICATIONS

This application claims the benefit of U.S. Application No. 61/228,101, filed July 23, 2009.

### TECHNICAL FIELD

The present invention relates generally to an integrated diabetes management system, and more particularly, to the use of exogenous data to predict alarms and to manage glucose levels.

### BACKGROUND

Glucose monitoring systems for patients afflicted with diabetes may incorporate various functionalities, including a capability to project or predict alarms to warn patients and/or provide information related to expected glucose levels, for example. Various factors may affect glucose levels; however, glucose monitoring systems generally only have access to certain types of information and factors (i.e., the monitored information). Thus, the projected alarms generated by such glucose monitoring systems are based on limited data and, although quite helpful, may be less reliable than if additional relevant information and factors were taken into account in projecting the alarms.

In a continuous glucose management ("CGM") system, it is possible to predict if the glucose is going to cross a hypoglycemic or hyperglycemic threshold in the future by using the CGM data. One way to do this it so estimate the rate-of-change of the glucose and project from the latest glucose point to some time in the future. While this projected alarm is helpful, there can be a significant number of false alarms and misdetections. These often occur when the glucose level of the patient changes direction, which often occurs. These changes are caused by physiological effects (the body's production if insulin), insulin boluses, meal intake, exercise, and other causes.

In the past, continuous glucose monitoring and continuous insulin delivery are accomplished by different pieces of hardware devices that do not share data. Each device provides real time management tools for diabetes and insulin delivery respectively. With the convergence of continuous glucose monitoring and insulin pumps, real-time management tools could be developed that will enhance the existing tools and provide new real-time management functionalities that did not exist before.

For example, the FreeStyle Navigator^{®} system from Abbott Diabetes Inc, Alameda, California, a continuous glucose monitor, provides a projected low glucose (hypoglycemia) alarm function using the trend of the glucose profile and the rate of change of glucose to predict when the glucose reading would fall below the low threshold that can be set by the user. The user can set the alarm sensitivity to receive a warning of up to thirty minutes prior to the low glucose event. With the addition of insulin delivery data, for example, the "insulin on board" information from the insulin pump, then we would be able to enhance the reliability of the projected low glucose alarm to be provided earlier and provide a tool for the user to figure out the amount of carbohydrates to take to prevent the low blood glucose from occurring.

As used herein, the term "exogenous" data is meant to encompass measurements other than glucose measurements.

On the other hand, many therapy parameters that govern the real time bolus decision using the insulin pump can be better adjusted and refined with the availability of the continuous glucose information. For example, many smart pumps today provide a way to calculate the amount of insulin to cover a food or meal event through the use of the carbohydrate ratio (also referred to as "carb ratio" herein) and the bolus calculator. However, the precise carbohydrate ratio to use is an empirically derived number. With the continuous glucose data available, the "accuracy" of the carbohydrate ratio used for a food bolus calculation may be assessed in real time to provide adjustment guidance for refining the carbohydrate ratio for use in the subsequent food or meal event.

In WO 2009/049252 A1, there is described methods and apparatus for determining analyte concentration in a sample such as bodily fluid.

In US 6,923,763 B1, there is described a method which utilizes blood glucose sampling, insulin infusion/injection records, heart rate information and heart rate variability information to estimate BG in the near future and to estimate of the risk of the onset of hypoglycemia.

In US 2008/300572 A1, there is described a monitor device for a fluid infusion system and its operating, display, and data processing characteristics.

Hence those of skill in the art have recognized a need for increased reliability of projected glucose alarms. Those skilled in the art have also recognized the need for the instant or near-instant incorporation of exogenous data to further increase the reliability and effectiveness of projected alarms. A further need has been recognized for providing tools to more accurately control glucose levels; and further, those skilled in the art have identified a need for the use of exogenous data in fine tuning the management of a diabetic patient's glucose control. The present invention fulfills these needs and others.

### SUMMARY OF THE INVENTION

Various aspects and embodiments of the invention are set out in the appended claims 1 - 15.

Briefly and in general terms, the invention is directed to a system and method for processing glucose level measurement data with exogenous data to result in more reliable recommended therapeutic response and to enable tuning of patient-specific insulin data.

The invention as defined in claim 1 relates to an integrated glucose monitoring system, comprising: a memory configured to store data relating to at least two glucose measurements of a user, a target glucose level, and exogenous data, wherein the exogenous data comprises at least one of insulin on board data, meal data, prior carbohydrate intake data or carbohydrate ratio data, wherein the at least two glucose measurements of the user are at different time points t1 and t2; a user interface comprising a display; a communication module configured to communicate with an insulin pump to acquire insulin delivery data; and a processor comprising computer-executable instructions to: determine a rate of change between the at least two glucose measurements of the user; determine a projected glucose level at a future time t3 based on at least the two glucose measurements, the determined rate of change, the exogenous data, and the acquired insulin delivery data; and provide a recommended therapeutic response if the projected glucose level at future time t3 exceeds the target glucose level.

Independent claims 9 and 15 define a corresponding method and non-transitory computer-readable medium having corresponding instructions stored thereon.

The dependent claims define preferred embodiments.

### BRIEF DESCRIPTION OF THE DRAWINGS

Various features and advantages of the disclosure will become more apparent by the following detailed description of several embodiments thereof with reference to the attached drawings, of which:
FIG. 1 is a graph providing the change in patient glucose resulting from changes in basal rate over time showing in particular the increase in glucose with a lowered basal rate and a decrease in glucose with a higher basal rate;
FIG. 2 is a graph that displays patient glucose versus time related to changes in carb ratio and carbohydrate bolus, according to an embodiment;
FIG. 3 is a graph that displays the effect of more accurate projected alarms when an unexpected rise in glucose occurs taking the glucose to a hyperglycemic state, showing the effect that early insulin delivery can have;
FIG. 4 is a graph of glucose versus time that illustrates the result of the delivery of a correction bolus when insulin sensitivity is considered;
FIG. 5 illustrates a block diagram of an integrated continuous glucose monitoring and insulin pump system, according to an embodiment in which more reliable projected alarms are provided and in which profiles of continuous glucose monitoring data are produced for tuning of exogenous patient-specific insulin data for more accurate control over a patient's glucose levels;
FIG. 6 is a flow chart illustrating a method for managing glucose levels, projections, and alarms related to glucose levels, according to an embodiment; and
FIG. 7 is a flow chart showing a profile recording feature in accordance with aspects of the invention in which profiles are formed of continuous glucose data of the patient and are used to determine fine tuning of patient-specific insulin values to achieve better control over a patient's glucose levels.

### DETAILED DESCRIPTION OF THE PREFERRED EMBODIMENTS

Reference will now be made in detail to various embodiments of the invention, examples of which are illustrated in the accompanying drawings, wherein like reference numerals refer to like or corresponding elements throughout. While the embodiments are described with detailed construction and elements to assist in a comprehensive understanding of the various applications and advantages of the embodiments, it should be apparent however that the embodiments can be carried out without those specifically detailed particulars. Also, well-known functions or constructions will not be described in detail so as to avoid obscuring the description with unnecessary detail. It should be also noted that in the drawings, the dimensions of the features are not intended to be to true scale and may be exaggerated for the sake of allowing greater understanding.

An integrated continuous glucose monitoring (CGM) and medication delivery system, such as an insulin pump, is highly advantageous as two types of information (i.e., continuous glucose monitoring information (e.g., glucose trend and profile information) and continuous insulin delivery information from the medication delivery system) may be combined for various calculations, predictions, and analyses useful in managing a person's diabetes. Examples of the calculations, predictions, and analyses include, but are not limited to: a projected alarm for providing a warning for insulin excess and carbohydrate requirements; a temporary basal manager for managing basal rate reduction through temporary basal control; a basal rate tuner for adjusting basal rate using continuous glucose information; a carbohydrate ratio tuner for adjusting carbohydrate ratio used in a bolus calculator for administering food bolus; and a correction factor tuner for adjusting insulin sensitivity in a bolus calculator for administering a correction bolus.

As used herein, the term "project" or "projected" is synonymous with "predict" or "predicted" and "forecast" or "forecasted."

As noted above, information related to meals and insulin delivery may affect a projected alarm. For example, if a projected hyperglycemia alarm were about to occur, but information related to a recent insulin bolus delivery were provided, then the projected alarm may be desirably delayed as it may be unnecessary due to the effect that the recent bolus delivery will have.

More specifically, a CGM system may predict if a glucose level is going to cross a hypoglycemic or hyperglycemic threshold based upon monitored glucose data, such as rate-of-change and the projected glucose level. However, the glucose level may change direction due to, for example, physiological effects (e.g., production of insulin), bolus, meal intake, exercise, and other factors. The change in direction may have the effect of the glucose level not crossing a threshold, thus making a projected alarm unnecessary. Thus, taking into account the factors noted above (e.g., physiological effects (e.g., production of insulin), bolus, meal intake, exercise, and other factors), it can then be determined if a projected alarm is indeed necessary, thereby increasing the efficacy of the projected alarm in its predictive capability features.

Examples of the increased reliability of the predictive capability features when insulin delivery and meal information are taken into account include the following: (i) if the projected alarm feature indicates that the hyperglycemic event was going to happen soon, but a bolus occurred ten minutes prior, the projected alarm may be cancelled; (ii) if a projected low glucose alarm was about to occur but a meal event was recently entered, the projected alarm may be cancelled; (iii) if a projected low glucose alarm was about to occur but an insulin bolus was recently given, then the projected alarm may be initiated immediately instead of waiting for the previously scheduled time; and (iv) if the presence of "exogenous" data, such as insulin on board (IOB), suggests that the patient is in an "insulin excess" state, then the projected alarm may be initiated when in the absence of the IOB information, such projected alarm would not be indicated.

To implement a projected alarm that provides a warning for insulin excess and to provide carbohydrate requirements, the following factors may by utilized to determine when a patient's glucose level will fall below an acceptable limit: current glucose level, insulin on board ("IOB"), insulin sensitivity, carbohydrate ratio, and duration of insulin action. Other factors may also be taken into account. As used herein, the "carbohydrate ratio" refers to the amount of carbohydrates required for each unit of insulin.

The two scenarios in Table 1 below may be used to illustrate the projected alarm feature, according to an embodiment, in which a patient's current glucose level is 173 mg/dL. A response in each scenario may differ depending on the availability of IOB information. The presence of IOB information can enhance the time horizon of a low glucose (hypoglycemia) projected alarm over one that is based solely on using glucose rate of change information.

**Table 1**

| | Scenario 1 | Scenario 2 |
|---|---|---|
| Current glucose | 173 mg/dL | 173 mg/dL |
| Insulin on board (IOB) | NO IOB information | 4.6 units |
| Carb ratio | 12 grams | 12 grams |
| Target glucose level | 100 mg/dL | 100 mg/dL |
| Insulin sensitivity | 45.6 mg/L | 45.6 mg/L |
| Alarm information | No alarm is indicated. | Indicate projected low glucose alarm. Glucose level will fall below low threshold of 70 mg/dL in about 2.5 hours. |
| | | This calculation is based on insulin action profile, insulin action time, and insulin sensitivity. |
| | | In this example, glucose will fall below 70 |
| | | mg/dL when 2.26 units of insulin are used up. Assume a linear insulin action profile for the purpose of the illustration; this is about (~49% of current insulin on board amount) 2.5 hours if time of insulin action is set at 5 hours. |
| Possible Recommendation | Patient needs 1.6 units of insulin bolus. | In addition to initiating a projected low glucose alarm, provide a potential avoidance strategy: needs 36 g of carbohydrates to cover the excess insulin. |

With scenario 2 illustrated above in Table 1, an enhanced projected hypoglycemic alarm based on the use of the exogenous insulin on board, and with the availability of personalized therapy parameters like insulin sensitivity, carb ratio, and target BG, may signal to the patient that within approximately 2.5 hours, a low glucose level will be reached.

According to another embodiment, an "excess insulin manager" can provide a recommendation to avoid the future low glucose level depending on the type of information available to the system. For example, in Table 1, Scenario 2, with the availability of the particular patient's carb ratio, the excess insulin manager can provide the recommendation to take 36g of carbohydrates now.

A temporary basal approach, according to another embodiment, operates to determine a basal reduction necessary to compensate for excess insulin on board, when additional access and control of the insulin delivery rates are available. With the same example in Scenario 2 of Table 1, the "excess insulin manager" can provide a different option to deal with excess insulin by reducing the future basal insulin delivery by programming a temporary basal insulin reduction. This alternative may be preferred because the user would not need to eat additional carbohydrates, which tend to add weight to the patient. Furthermore, the "excess insulin manager" could provide the means for the user to take both options to offset the excess insulin; i.e., take additional carbohydrates and make a temporary basal reduction.

Table 2 below shows that multiple recommendations may be possible after the initiation of the low alarm depending on access to insulin delivery information and control. In Table 2, Scenario 2 is the same as Scenario 2 in Table 1 except for the "possible recommendation" in that in Table 2, multiple recommendations are provided due to the presence of the basal rate information.

**Table 2**

| | Scenario 2 | Scenario 3 |
|---|---|---|
| Current glucose | 173 mg/dL | 173 mg/dL |
| Insulin on board (IOB) | 4.6 units | 4.6 units |
| Carb ratio | 12 grams | 12 grams |
| Target glucose level | 100 mg/dL | 100 mg/dL |
| Insulin sensitivity | 45.6 mg/L | 45.6 mg/dL |
| Basal insulin delivery rate | | |
| Alarm information | Indicate projected low glucose alarm. Glucose level will fall below low threshold of 70 mg/dL in about 2.5 hours. | Indicate projected low glucose alarm. Glucose level will fall below low threshold of 70 mg/dL in about 2.5 hours. |
| | This calculation is based on insulin action profile, insulin action time, and insulin sensitivity. | This calculation is based on insulin action profile, insulin action time, and insulin sensitivity. |
| | In this example, glucose will fall below 70 mg/dL | In this example, glucose will fall below 70 mg/dL |
| | when 2.26 units of insulin is used up. Assume a | when 2.26 units of insulin is used up. Assume a |
| | linear insulin action profile for the purpose of the illustration; this is about (~49% of current insulin on board amount) ~2.5 hours if time of insulin action is set at 5 hours. | linear insulin action profile for the purpose of the illustration; this is about (~49% of current insulin on board amount) ~2.5 hours if time of insulin action is set at 5 hours. |
| Recommendation | No specific insulin rate information: | With additional basal rate information: |
| | Needs 36 g of carbohydrates to cover the excess insulin. | a) needs 36 g of carbohydrates to cover the excess insulin |
| | | b) reduces future basal delivery to offset the excess insulin (2.34 units). Specific temp basal rate and time can be provided to facilitate quick setting of the rate on an integrate pump. |
| | | c) combination of both - wizard like process can guide the user through determining how much carbs to take and how much reduction in basal to make into the future. |

According to another embodiment, the "excess insulin manager" could be initiated by the user, instead of being initiated by the occurrence of a projected hypoglycemia alarm. The user may choose to turn off the projected alarm and use the "excess insulin manager" features on-demand. An example would be where the user wants to check for excess insulin right before going to bed so that he or she can take appropriate measures to avoid nocturnal hypoglycemia.

The production of continuous glucose data by a CGM system offers the ability to fine tune various glucose management factors to permit more accurate control of a patient's glucose levels. For patients having an insulin pump, a basal rate of insulin delivery is typically assigned for the purpose of maintaining constant control over the patient's glucose level. Additionally, a carb ratio, mentioned above, is determined for each patient based on that patient's characteristics, which is used to determine the amount of carbohydrates that will cover, or neutralize, one unit of insulin in that particular patient. Insulin amounts and calculations of carbohydrate amounts for this patient are determined by his or her carb ratio. Also, the individual patient's sensitivity to insulin or "correction factor" is determined and is used to calculate the speed with which insulin affects the patient, also a factor in determining when and how much insulin to deliver to that patient. To provide further control over the patient's glucose, a profile recording feature is provided.

The "profile recording feature" that can be enabled by the user to record the glucose data from the CGM system as a particular "profile." User action may be used to set the start and end times of the profile recording or the user may "tag" an event in which case the processor will initiate and terminate the data recording and label that recorded data as a particular user profile. For example, the profile recording may require user input of the start time and the end time. The start time could be manually entered, or automatically entered if it corresponds to the time of certain user or device action, such as the start of a meal bolus, the start of a correction bolus, etc. The end time could be manually entered by the user or it could correspond to the completion of certain user or device action. These profiles can be categorized by the type of user actions, analyzed, and displayed in reports for use in fine tuning glucose management factors. A specific rule engine can be implemented to provide actionable recommendations to the user based on the output of the recorded profiles.

Some examples of rules that may be implemented are: 1) rule for persistent/recurring profile, e.g., require a minimum numbers of profiles, N, all of which must be greater than the target or all of which must be less than the target by certain amount X before a recommendation for a change in a factor can be given; 2) rule for usable profile for analysis; e.g., only a test measured within the last two weeks can be used for analysis for an insulin recommendation; data that is too old could be irrelevant now; 3) rule for refreshing the profile queue; more profiles must be entered into the queue after every insulin change that has taken place, or after X amount of time has elapsed since the first recording; and 4) rule for safety, that is, only allow an increase in basal insulin of ten percent at a time and set the maximum incremental change possible for the carb ratio and the correction factor.

The above rule engine settings can be set by the user or by the health care provider ("HCP") depending on the sophistication of the user. Also, the user's diabetes management devices may have possible security and lock out features so that only the HCP can set the rules without allowing the user to change them.

According to one embodiment, a "skip-meal test" feature is provide to the user to allow the user to mark the CGM data during the meal that is skipped for the purpose of fine-tuning the basal rate. Upon a user's initiating the "skip-meal test," the user will be given a chance to choose the start time of the skip-meal test (either the at the time when the "skip-meal test" is initiated on the user's interface ("UI"), or the user will be given the means to set a time prior to when the "skip-meal test" is initiated). The system will then record the CGM data starting from the user-indicated start time to form a skip-meal test profile. The recording of this CGM profile will end upon a certain user indication through the device UI, a spike in the CGM signal, or a certain device event (e.g., a meal bolus).

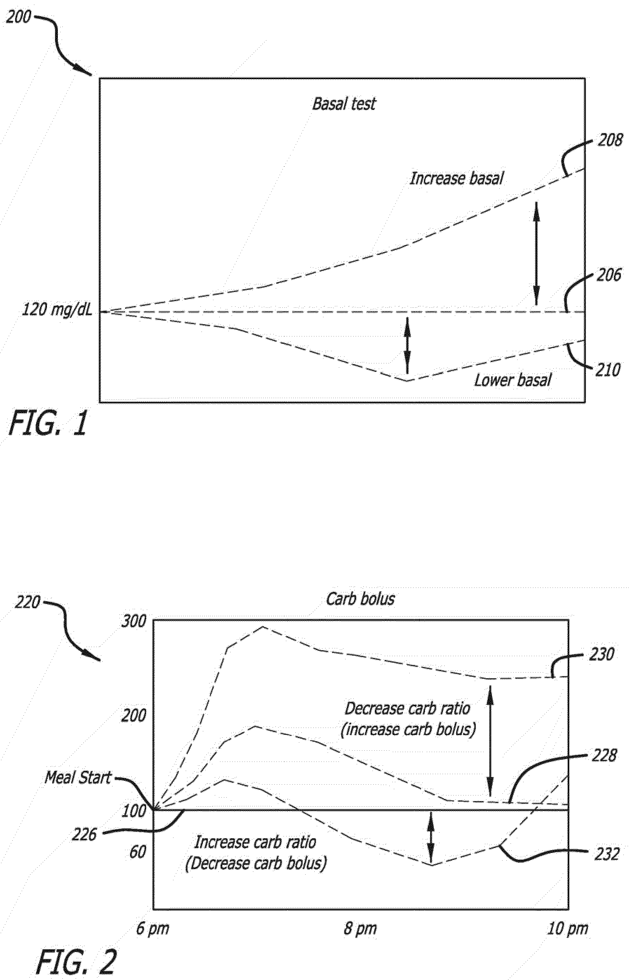

A "skip meal profile" report similar to FIG. 1 may be used to analyze and visualize the recorded "skip-meal" profiles. With reference to FIG. 1 ("Basal Rate Tuner"), a graph 200 is provided to help the user decide whether a basal rate adjustment may be needed based on the recorded "skip meal profiles." The left vertical axis provides the glucose level in mg/dL in this embodiment while the bottom horizontal axis provides time, starting with the start time of the individual profile recording. The 120 mg/dL line 206 indicates the target glucose level. The dashed line 208 above that level indicates a higher glucose level than target, which may indicate that an increase in basal rate should be effected. The dashed line 210 below target 206 indicates a low glucose and that perhaps the basal rate should be lowered. If persistent pattern is observed in the skip-meal profiles over a certain time, an appropriate action can be recommended by the "basal rate tuner" to fine tune the basal rate to get it closer to the target level 206. However, if a persistent pattern is not observed over time, such a recommendation may not be made since the data does not clearly indicate that a continuous problem exists.

A "carb tuner" is also provided in which a "meal test" feature is provided to the user. With this feature, the meal test allows the user to start recording the CGM data to form a meal test profile after a "meal event," that profile representing the glucose response for that meal. Typically, a patient takes an insulin bolus prior to the start of a meal to manage the likely increasing spike in glucose level that eating a meal causes. That meal bolus for the patient is calculated using the patient's carb ratio. The meal test feature permits the patient to ascertain whether his or her carb ration needs tuning.

The start of the recording of CGM data is based on a user prompt, a user entered meal event, or a device-driven event such as a meal bolus. The end of the recording of CGM data for that profile is based on user prompt or a pump device related parameter, such as the insulin action time. Appropriate meal bolus parameters such as the carb ratio will be saved as an attribute that will be associated with the profile. Additional user-entered or device related-tags, such as food description and meal type (breakfast, lunch, dinner, snack, etc.) can also be added as attributes that can be associated with the recorded profile.

A "meal test' report, similar to that shown in FIG. 2, may be used to analyze and visualize the recorded "meal test" profiles for the purpose of fine tuning the user's carbohydrate ratio used in a bolus calculator for recommending food bolus amounts at meal times in an integrated CGM and insulin pump system.

With further reference to FIG. 2 ("Carbohydrate Ratio Tuner"), a graph 220 provides information related to the user's carbohydrate ratio. The left vertical axis provides the user's glucose level while the bottom horizontal axis provides time. The graph illustrates when to increase the patient's carbohydrate ratio (and decrease the carbohydrate bolus) and when to decrease the patient's carbohydrate ratio (and increase the carbohydrate bolus). The 100 mg/dL solid line glucose level 226 is the target glucose level and the effects of a meal, taken at 6 pm are seen with the three representative lines. A correct carb ratio is shown with the middle dashed line 228 showing a modest increase in glucose following the mean with a drop back to near target glucose level at about 9 pm. However, the upper dashed line 230 shows an undesirable increase in glucose indicating a carb ration that is too high. The line 230 may indicate that a decrease in the carb ratio should be determined for this patient, depending on whether a persistent pattern exists over time.

The lower dashed line 232 indicates that the glucose level dropped much lower than desired after the meal thus indicating that the carb ratio is too low and should be raised. As with the highest glucose line on the figure, i.e., line 230, a change in the carb ratio for this patient may be needed depending on whether a persistent pattern appears over a period of time. Such a persistent pattern in glucose levels, as determined by the integrated CGM and insulin pump system, is utilized to calculate a change recommendation in carb ratio for the patient. Additional data labels can be used to show the various profile attributes to interpretation.

In another embodiment, a collection of "meal test profiles" may be accessed by the patient prior to meal bolus events. A "meal bolus profile" queue may be implemented as part of the carb ratio selector during a meal bolus calculation process, in which the patient can select the carb ratio based on the previously recorded profile. The queue may indicate a carb ratio to use for meal bolus events. The results in the queue may be standardized, according to an embodiment, to group together multiple meal bolus events so that a general modification recommendation may be generated. Such collected profiles may be characterized by a particular meal, such as breakfast or other, and may be further characterized by the type of food to be eaten for that meal. The patient may then want to select the profile that provided the best glucose control for that prior event and have that previous carb ratio applied to the present meal event.

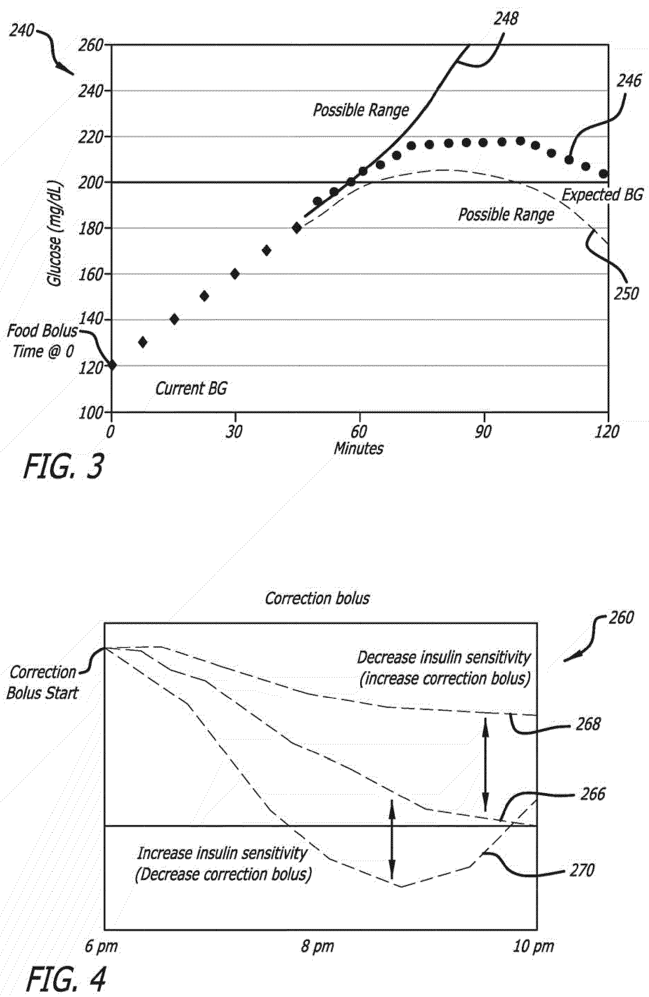

Moreover, the "meal test profile" and "carbohydrate ratio tuner" features may provide the means to add a warning mechanism for an under-bolus alarm in which under-dosing of food bolus insulin occurs if the food bolus profile deviates from an expected pattern based on the selected recorded "meal test profile". For example and with reference to FIG. 3 ("Carbohydrate Ratio Tuner - Early Warning" with glucose level at the vertical left axis and time at the bottom horizontal axis), a graph 240 is provided that indicates an expected glucose level 246, as well as possible ranges of the expected glucose level based on previously recorded profiles. A meal is consumed at 0 minutes with a glucose level of 120 mg/dL. A possible range is shown with an upper solid line 248 and the lower dashed line 250. The expected glucose level 246 is shown with the dotted line. The under-bolus alarm may be generated to indicate a need for patient intervention even if a hyperglycemia alarm is not triggered.

A third tuner is the insulin sensitivity factor or correction factor. A "correction bolus test" feature is provided to allow the user to record the CGM data profile after a correction bolus delivery for the purpose of fine-tuning the insulin sensitivity factor that may be implemented in the integrated CGM and insulin pump system. The start of the recording is based on a user prompt, a user entered correction bolus event, or a device-driven event such as a correction bolus. The end of the recording is based on a user prompt or a pump-device related parameter, such as the insulin action time. Appropriate correction bolus parameters, such as the insulin sensitivity factor, will be saved as an attribute that will be associated with the correction bolus profile. Additional user-entered or device-related tags (e.g., time of day) can also be added to the stored data as attributes that can be associated with the recorded profile.

A "correction bolus test' report, similar to FIG. 4, may be used to analyze and visualize the recorded "meal test" profiles for the purpose of tuning the carbohydrate ratio used in a bolus calculator for recommending food bolus amounts in an integrated CGM and insulin pump system.

With reference to FIG. 4 ("Insulin Sensitivity Tuner"), correction bolus information is displayed in graph 260, indicating a need to either increase insulin sensitivity (decrease correction bolus) or decrease insulin sensitivity (increase correction bolus). The glucose level is shown as the vertical left axis while once again time is shown as the bottom horizontal axis. In this case, all the "correction test profiles" with an insulin bolus delivery at 6 pm are plotted in a representative plot to illustrate the possible action based on the representative pattern of the profiles. The correct insulin sensitivity is shown as the middle dashed line 266. If the "correction test profiles" show a persistent pattern in regard to the upper dashed line 268 or the lower dashed line 270, appropriate actionable recommendations can be suggested to the user.

In yet another embodiment, these "correction bolus test" profiles related to insulin sensitivity adjustments may conveniently be provided prior to correction bolus events. The previously recorded profiles may be placed in a "correction bolus profile" queue for access by a patient. The results of the queue may be standardized, according to an embodiment, to group together multiple bolus results for a general modification recommendation for an upcoming correction bolus event. Should the patient experience a situation identical or similar to one of the profiles, he or she may select a profile from the queue for use of that sensitivity with the present situation.

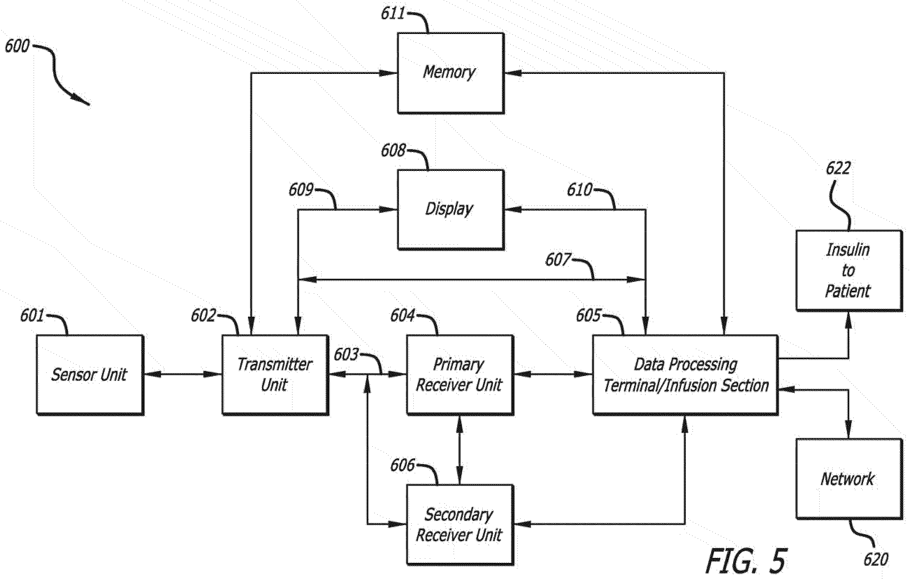

With reference now to FIG. 5, a block diagram of an integrated CGM and insulin pump system 600, according to an embodiment, is illustrated. The integrated CGM and insulin pump system 600 may operate to manage alarm projections and alarms related to glucose levels. The system is also configured and programmed to produce profiles from CGM data to be used in tuning certain glucose management factors, as discussed above.

The system 600 may include, according to an embodiment, a glucose monitoring and management portion as well as an insulin infusion pump that stores or otherwise acts upon data relating to glucose measurements, carbohydrate intake values, and other data of interest in diabetes management.

The system 600 may include a glucose sensor 601, a transmitter unit 602 coupled to the sensor 601, and a primary receiver unit 604 which is configured to communicate with the transmitter unit 602 via a communication link 603. Those of ordinary skill in the art will readily recognize that the sensor represented as element 601 may include a drug delivery device, such as an insulin infusion system which includes a transmitter and, if so, that the principles of data preservation and transfer disclosed herein would apply as well to such a system. Therefore, system 600 as depicted in FIG. 5 will be understood to be representative rather than limiting of the arrangements of medical data receivers and transmitters with which the present invention may be used.

The primary receiver unit 604 may be configured to receive data from the transmitter unit 602 and may be further configured to transmit data to a data processing terminal/infusion section 605 for evaluating the data received by the primary receiver unit 604. Moreover, the data processing terminal/infusion section 605 in one embodiment may be configured to receive data directly from the transmitter unit 602 via a communication link 607 which may optionally be configured for bi-directional communication.

Also shown in FIG. 5 is a secondary receiver unit 606 which is operatively coupled to the communication link 603 and configured to receive data transmitted from the transmitter unit 602. Moreover, as shown in FIG. 5, the secondary receiver unit 606 is configured to communicate with the primary receiver unit 604 as well as the data processing terminal/infusion section 605. The secondary receiver unit 606 may be configured for bi-directional wireless communication with each of the primary receiver unit 604 and the data processing terminal/infusion section 605. As discussed in further detail below, in one embodiment, the secondary receiver unit 606 may be configured to include a limited number of functions and features as compared with the primary receiver unit 604. As such, the secondary receiver unit 606 may be configured substantially in a smaller compact housing, for example. Alternatively, the secondary receiver unit 606 may be configured with the same or substantially similar functionality as the primary receiver unit 604 and may be configured to be used in conjunction with a docking cradle unit for placement by bedside, for night time monitoring, for example, and/or a bi-directional communication device.

Only one sensor 601, transmitter unit 602, communication link 603, and data processing terminal/infusion section 605 are shown in the integrated CGM and insulin pump system 600 illustrated in FIG. 5. However, the system 600 may include one or more sensors 601, transmitter units 602, communication links 603, and data processing terminal/infusion sections 605. In a multi-component environment, each device is configured to be uniquely identified by each of the other devices in the system so that communication conflict is readily resolved between the various components within the system 600.

In an embodiment, the sensor 601 is physically positioned in or on the body of a user whose analyte (e.g., glucose) level is being monitored. The sensor 601 may be configured to continuously sample the analyte level of the user and convert the sampled analyte level into a corresponding data signal for transmission by the transmitter unit 602. In one embodiment, the transmitter unit 602 is physically coupled to the sensor 601 so that both devices are positioned on the user's body, with at least a portion of the analyte sensor 60 I positioned transcutaneously under the skin layer of the user. The transmitter unit 602 performs data processing such as filtering and encoding on data signals, each of which corresponds to a sampled analyte level of the user, for transmission to the primary receiver unit 604 via the communication link 603.

In an embodiment, the system 600 is configured as a one-way RF communication path from the transmitter unit 602 to the primary receiver unit 604. In such embodiment, the transmitter unit 602 transmits the sampled data signals received from the sensor 601 without acknowledgement from the primary receiver unit 604 that the transmitted sampled data signals have been received. For example, the transmitter unit 602 may be configured to transmit the encoded sampled data signals at a fixed rate (e.g., at one minute intervals) after the completion of the initial power on procedure. Likewise, the primary receiver unit 604 may be configured to detect such transmitted encoded sampled data signals at predetermined time intervals. Alternatively, the integrated CGM and insulin pump system 600 may be configured with a bi-directional RF (or otherwise) communication between the transmitter unit 602 and the primary receiver unit 604.

In operation, upon completing a power-on procedure, the primary receiver unit 604 is configured to detect the presence of the transmitter unit 602 within its range based on, for example, the strength of the detected data signals received from the transmitter unit 602 or a predetermined transmitter identification information. Upon successful synchronization with the corresponding transmitter unit 602, the primary receiver unit 604 is configured to begin receiving from the transmitter unit 602 data signals corresponding to the user's analyte level as detected by the sensor 601. More specifically, the primary receiver unit 604 in an embodiment is configured to perform synchronized time hopping with the corresponding synchronized transmitter unit 602 via the communication link 603 to obtain the user's detected analyte level.

Referring again to FIG. 5, the data processing terminal/infusion section 605 may include, as examples, a personal computer, a portable computer such as a laptop or a handheld device (e.g., personal digital assistants (PDAs)), and the like, each of which may be configured for data communication with the receiver via a wired or a wireless connection. The data processing terminal/infusion section 605 includes a processor that includes computer-executable instructions for performing various functions and processing related to, for example, data transmitted and received within the system 600. Additionally, the data processing terminal/infusion section 605 may further be connected to a data network 620 for storing, retrieving, and updating data corresponding to the detected analyte level of the user, for example. Other types of data and information may also be stored, retrieved, and updated.

The data processing terminal/infusion section 605 may include an infusion device such as an insulin infusion pump or the like, which may be configured to administer insulin to a patient 622, and which may be configured to communicate with the receiver unit 604 for receiving, among others, the measured analyte level. Alternatively, the receiver unit 604 may be configured to integrate an infusion device therein so that the receiver unit 604 is configured to administer insulin therapy to patients, for example, for administering and modifying basal profiles, as well as for determining appropriate boluses for administration based on, among others, the detected analyte levels received from the sensor 601 through the transmitter unit 602.

Additionally, the transmitter unit 602, the primary receiver unit 604, and the data processing terminal/infusion section 605 may each be configured for bi-directional wireless communication such that each of the transmitter unit 602, the primary receiver unit 604, and the data processing terminal/infusion section 605 may be configured to communicate (that is, transmit data to and receive data from) with each other via the wireless communication link 603. More specifically, the data processing terminal/infusion section 605 may in an embodiment be configured to receive data directly from the transmitter unit 602 via the communication link 607, where the communication link 607, as described above, may be configured for bi-directional communication.

The data processing terminal/infusion section 605 which may include an insulin pump, may be configured to receive the analyte signals from the sensor 601 through the transmitter unit 602, and thus, incorporate the functions of the receiver 604 including data processing for managing the patient's insulin therapy and analyte monitoring. In an embodiment, the communication link 603 may include one or more of an RF communication protocol, an infrared communication protocol, a Bluetooth enabled communication protocol, an 802.11x wireless communication protocol, or an equivalent wireless communication protocol which would allow secure, wireless communication of several units (for example, per HIPPA requirements) while avoiding potential data collision and interference.

Additional detailed description of a continuous analyte monitoring system and its various components including the functional descriptions of the transmitter are provided in US Patent No. 6,175,752 issued January 16, 2001 entitled "Analyte Monitoring Device and Methods of Use," in US Application No. 10/745,878 filed December 26, 2003 entitled "Continuous Glucose Monitoring System and Methods of Use," and in US Application No. 12/024,101 filed January 31, 2008 entitled "Method and System for Determining Analyte Levels," each of which is assigned to the Assignee of the present application.

With further reference to FIG. 5, a display 608 is provided as part of the integrated CGM and insulin pump system 600. According to an embodiment, the display 608 may be part of the data processing terminal/infusion section 605. For example, the display 608 may be a monitor of the data processing terminal/infusion section 605, which may be a personal computer or a portable computer. Alternatively, the display 608 may be a separate component coupled to the transmitter unit 602 and/or the data processing terminal/infusion section 605 respectively via communication links 609 and 610. The communication links 609 and 610, similar to the link 603 described above, may incorporate a wireless communication protocol for secure, wireless communication. Or, the communication links 609 and 610 may be directly or indirectly wired.

The display 608 may include a graphical user interface for displaying received and/or processed information to a patient or other user. The received and/or processed information may come from various and multiple sources, such as the sensor unit 601, the transmitter unit 602, and/or the data processing terminal/infusion section 605. For example, as described above, the transmitter unit 602 may be configured to receive data from the sensor unit 601, while the primary receiver unit 604 may be configured to receive data from the transmitter unit 602 and may be further configured to transmit data to the data processing terminal/infusion section 605 for evaluating the data received by the primary receiver unit 604. The evaluated data, as processed by the data processing terminal/infusion section 605, may then be displayed on the graphical user interface of the display 608, for example.

The evaluated data may be displayed in various forms and/or representations, such as charts, graphs, and/or tables. The evaluated data may simply be presented as a list or bulleted items on the display 608. According to an embodiment, the evaluated data may include graphs 200, 220, 240, and 260 as described above and as processed by the data processing terminal/infusion section 605 with both data from the sensor unit 601 and the insulin pump incorporated in the data processing terminal/infusion section 605.

With further reference to FIG. 5, the integrated CGM and insulin pump system 600 includes one or more memory components 611 configured to store various data and/or datasets. The one or more memory components 611 may be part of the data processing terminal/infusion section 605, and/or the one or more memory components 611 may be separate components residing, for example, in an external server or data network 620.

According to an embodiment, the memory component 611 stores data related at least to the following: measurements of a physiological glucose level in a patient (endogenous data); a target glucose level for the patient (exogenous data); and one or more medically relevant data points (exogenous data), such as insulin on board, insulin sensitivity, prior carbohydrate intake, basal rate of insulin, carb ratio, available insulin bolus, profiles of CGM data, and other. The exogenous data of a patient's basal rate, carb ratio, and insulin sensitivity are patient-specific and are related to insulin. These may also be referred to as exogenous patient-specific insulin values.

The measurements of a physiological glucose level in a patient may include two measurements taken at different time points *tl* and *t2,* for example. The measurements may be taken from the sensor unit 601 at predetermined time points *tl* and *t2* as established by the patient or another, such as a clinician, or other health care provider. The time points *tl* and *t2* may be provided to the system 600 through use of the graphical user interface on the display 608. Alternatively, the time points *tl* and *t2* may be randomly selected by the data processing terminal/infusion section 605 based upon monitored and/or processed criteria, for example. As an example, the physiological glucose level of the patient may be continuously monitored and processed, and the data processing terminal/infusion section 605 may select two or more of the monitored levels.

The target glucose level for the patient, also stored in the memory component 611, may be established by the patient or a clinician or other healthcare professional. The target glucose level may be provided to the system 600 through use of the graphical user interface on the display 608. Alternatively, the target glucose level may be sent to the system 600 from a remote server or data network 620.

The other medically relevant data points stored in the memory component 611 may be data that is monitored and/or otherwise processed by the integrated glucose monitoring and insulin pump system 600. For example, and as described in further detail above with respect to FIGS. 1-5, the data may be exogenous and related to insulin delivery and meal information and may include, but is not limited to, insulin on board, insulin sensitivity, prior carbohydrate intake, carb ratio, basal insulin rate, and available insulin bolus. Such exogenous data, along with the target glucose level may be input to the memory 611, processor 605 and other parts of the system through various means, one of which includes a keyboard that is connected with the memory 611 or the processor 605. Such data may also be received from a remote source through the network 620 which may be in wired or wireless contact with other sources.

The computer-executable instructions of the data processing terminal/infusion section 605 may operate to process data stored in the memory component 611. For example, the instructions may operate to determine a rate of change between the glucose level measurements and an expected glucose level at a future time point t3. According to an embodiment, the time point t3 may be up to thirty minutes after the time point *t2,* for example. The computer-executable instructions may be further configured to identify whether this expected glucose level is above or below the target glucose level established for the patient. According to an embodiment, the expected glucose level may be a function of the rate of change between the two glucose level measurements as well as the medically relevant data point or points.

The computer-executable instructions may be further configured to provide an alarm if a difference between the expected glucose level and the target glucose level exceeds a preset warning value. The preset warning value may be established by the patient, a clinician, or other health care provider and may be provided to the system 600 through a graphical user interface of the display 608 or through other means as discussed above. The alarm may be in various forms, such as a sound alarm that serves to be audibly delivered to the patient, a visual alarm provided via the display 608 through the graphical user interface, a vibratory alarm, an email or other message delivered to a device of the patient, or a combination thereof.

The data processing terminal/infusion section 605, through the computer-executable instructions, may be further configured to determine and provide a recommended change to one or more of the medically relevant data point or points or factors. Such a recommended change may include, for example, a therapeutic response, such as a particular insulin bolus, intake of a particular level of carbohydrates, and/or a change or changes to exogenous data factors, such as the basal insulin level, the carb ratio, or the insulin sensitivity. The recommended therapeutic response may be displayed on the display 608 for ease in recognition by the patient.

The alarm and/or the recommended change may take the form of, for example, the graphs 200, 220, 240, and 260 (FIGS. 1, 2, 3, and 4). Additionally, the alarm and/or recommended change may be in the form of tables, such as Table 1 and Table 2 provided above. Profiles of CGM data may also be provided for review and selection by the patient, as discussed above. The patient may review the profiles, the comparisons of the profiles that show a persistent pattern and may retune one of more of the exogenous data in accordance with a processor's analysis of those profiles. For example, the patient may lower his or her basal rate to be consistent with the results of the profiles of the basal test by making the change on a keyboard through programming of the processor 605 or by use of a different user interface, such as the graphical user interface provided on the display 608. On the other hand, a patient may select a stored profile similar to the present conditions and command the processor to apply the stored profile's attributes to management of the patient's diabetes presently. Further, the processor may perform the safety checks of any exogenous data changes at this time to be sure that requested data changes are not inconsistent with the safety rules.

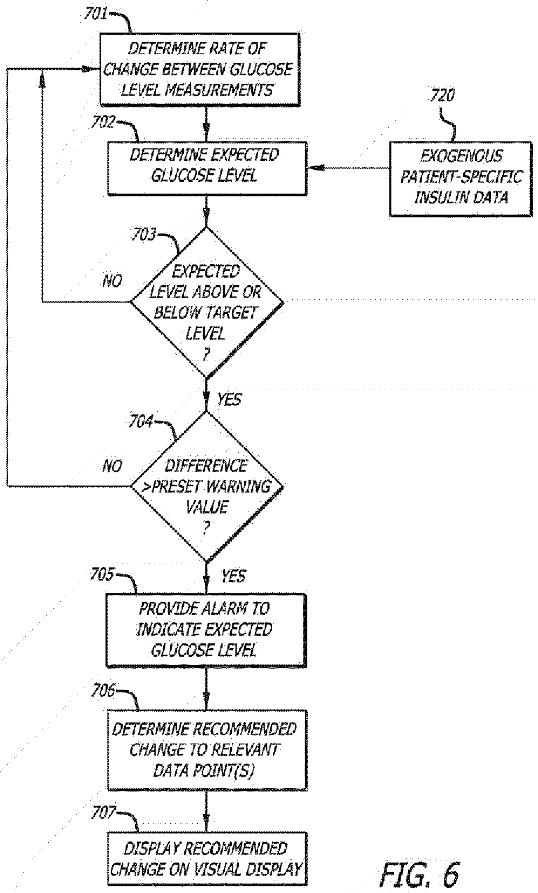

With reference to FIG. 6, a flow chart illustrates a method for managing projections and alarms related to glucose levels.

At 701, a rate of change between at least two glucose level measurements of a patient taken at different time points *tl* and *t2* is determined.

At 702, an expected glucose level at a future time point *t3* is determined, which may be a function of the rate of change between the at least two glucose level measurements and a function of at least one other medically relevant data point selected from exogenous data, such as the group of insulin on board, insulin sensitivity, prior carbohydrate intake, basal insulin, and available insulin bolus, for example. According to an embodiment, the time point *t3* may be up to thirty minutes after the time point *t2*. Other time intervals may be used, however, and the time point *t3* may be any amount of time following the time point *tl* and the time point *t2.*

At 703, a determination is made as to whether the expected glucose level at the time point *t3*, as determined at 702, is above or below a target glucose level established for the patient. If the expected glucose level is equal to the target glucose level or within a safe range, the process may continue back to 701 to determine a new rate of change between at least two glucose level measurements of the patient.

If it is determined at 703 that the expected glucose level is above or below the target glucose level or outside a safe range, a determination is made as to whether the difference between the expected glucose level and the target glucose level exceeds a preset warning value at 704, as may be established depending upon the patient and other factors. If the difference does not exceed the preset warning value, the process may continue back to 701 to determine a new rate of change between at least two glucose level measurements of the patient.

However, if the difference exceeds the preset warning value, an alarm is provided at 705 to warn the patient and/or clinician of the expected glucose level. The alarm may be one or more of a visual alarm, an audible alarm, a vibratory alarm, and a message sent to the patient and/or clinician. If the alarm is a visual alarm, the alarm may be provided on the graphical user interface of the display 608, for example.

At 706, a recommended change to one or more of the medically relevant data points is determined. The determination of the recommended change may include, for example, determining a therapeutic response, which may, include a particular insulin bolus, intake of a particular level of carbohydrates, temporary change to a basal insulin level, change carb ratio, or change insulin sensitivity. Other therapeutic or tuning responses may also be established and used.

At 707, the recommended change is displayed on a visual display, which may include a graphical user interface. For example, the recommended change may be provided on the graphical user interface.

An automatic response to an alarm may also be provided. For example, if an alarm is provided that is indicative of a very low glucose level, provision of an insulin bolus may be locked out at insulin pump system 600; e.g., by operation of a switch triggered in response to the alarm, preferably subject to manual override to ensure the availability of an insulin bolus if medically necessary.

According to embodiments of the present invention, methods are provided to increase reliability of projected alarms and reduce false alarms, which is highly desirable for improving the management of the user's medical condition. For example, in one embodiment a hyperglycemic projected alarm is provided in which false alarms are reduced. The method is provided as follows:
a) for every measurement of physiological glucose level in a patient performed by the CGM, the projected glucose is calculated based on the latest glucose reading, latest glucose trend estimate and projection time, which is generally a predetermined amount of time (e.g., 20 minutes), for example: projected glucose = current glucose + trend * projected time;
b) if the projected glucose exceeds a predetermined hyperglycemic threshold (e.g., 300 mg/dL), then proceed to (c); and
c) if an insulin bolus greater than a predetermined amount (e.g., 1 Unit) has not been delivered within a predetermined time (e.g., 20 minutes), then assert the projected alarm; otherwise, do not assert the projected alarm.

In another embodiment, a hypoglycemic projected alarm is provided in which reliability is increased and false alarms are reduced. For example, the method is provided as follows:
a) for every measurement of physiological glucose level in a patient performed by the COM, the projected glucose is calculated based on the latest glucose reading, latest glucose trend estimate and projection time, which is generally a predetermined amount of time (e.g., 20 minutes), for example: projected glucose = current glucose + trend * projected time;
b) if the projected glucose falls below a predetermined hypoglycemic threshold (e.g., 60 mg/dL), then proceed to (c); and
c) if a meal event greater than a predetermined amount (e.g., 1 Carb) has not been delivered within a predetermined time (e.g., 20 minutes), then assert the projected alarm; otherwise do not assert the projected alarm.

In related embodiments, hyperglycemic and hypoglycemic alarms similar to those described above are contemplated in which (a) and (b) compare the current glucose level in a patient to a predetermined threshold. In other related embodiments, (c) of the above hyperglycemic and hypoglycemic alarms may be modified such that even if the event (e.g., meal or insulin bolus event) occurs within the predetermined time, if the projected glucose level exceeds a second, more extreme threshold, the alarm is asserted. One of skill in the art would understand that all predetermined parameters (e.g., predetermined amounts and times) may be selectable by the patient or user:
Varying degrees of complexity are contemplated, for the above projected hyperglycemic and hypoglycemic alarms including elaborate model-based projected alarm as described further below. Accordingly, in yet another embodiment of the present invention, a model-based projected alarm is provided in which reliability is increased and false alarms are reduced. The method is provided as follows:
a) for every measurement of physiological glucose level in a patient performed by the CGM, the projected glucose is calculated based on one or more glucose readings; projection time (predetermined, e.g., 20 minutes); an exogenous parameter or parameters, for example one or more insulin/meal/exercise information parameters (e.g., bolus or meal amount and relative time of occurrence); and/or model parameters such as insulin sensitivity, insulin action time, carbohydrate ratio, carb uptake time, basal rate, or the like; and
b) if the projected glucose exceeds a predetermined hyperglycemic alarm threshold (e.g., 300 mg/dL), or falls below the hypoglycemic alarm threshold (e.g., 60 mg/dL), then the appropriate projected alarm is asserted.

In addition to potentially reducing the incidence of false alarms and thereby making projected alarms more reliable, the projected alarm may actually become more responsive with the additional inputs of meals and insulin; that is, the alarm may occur sooner than if these additional inputs were absent. For example, a patient's glucose may be at 100 mg/dL and trending downward, but not fast enough to alone trip the projected hypoglycemic alarm at this point in time. However, if a bolus occurs and is included in the projected alarm computation, the model may predict a more accurate projected glucose that will trip the projected alarm at this point in time. As discussed above, one of skill in the art would understand that all predetermined parameters described may be defined by the patient or user.

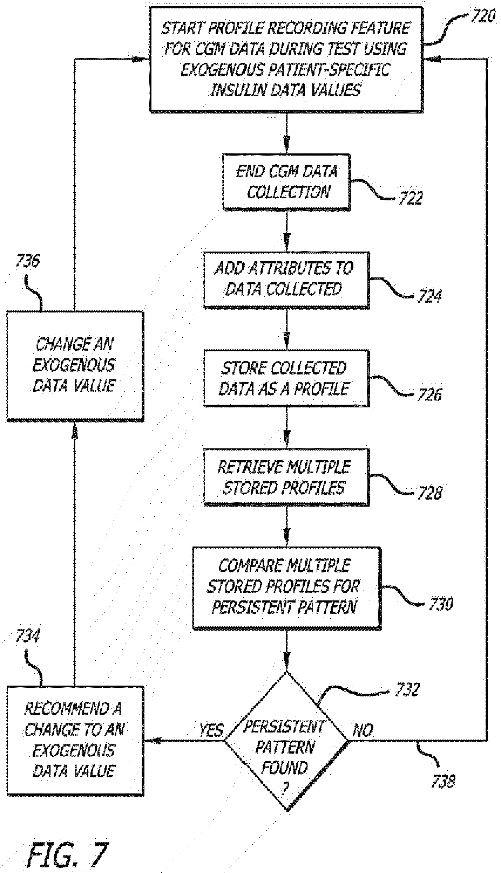

Turning now to FIG. 7, there is shown a flow chart of the process of forming and analyzing profiles of CGM data for "tuning" patient-specific insulin data values, such as the patient's basal delivery rate of insulin, the patient's insulin sensitivity, and the patient's carb ratio, all of which are used by the patient in the delivery of carbohydrates and insulin to control the patient's glucose levels. These tuners are discussed above.

Referring in detail to FIG. 7, the first step is one in which the recording of CGM data is started during a test or other selected time period 720, such as a basal test, skip-meal test, meal-test, or other. The means of starting the recording of the data may be a manual start signal from the patient or an automatic start control as discussed above. At the end of the test period or when sufficient CGM data has been collected, the recording feature is ended 722 either manually by the patient, such as by entering a stop signal at a keyboard, or by an automatic end recording feature, as discussed above. In certain cases, attributes are added to the recorded data file 724. As discussed above, such attributes may be varied but in one example, they may take the form of tagging the recorded CGM data as being "breakfast" data, or "high fat meal" data, or "post-prandial" data or others. Such data tagging can be highly useful in locating profiles of CGM data of similar past events that are stored in the memory 611 (FIG. 5). For example when a patient intends to consume a meal similar to one recently consumed, and in which the patient's glucose was successfully controlled to the desired safe range, the patient may desire to locate the stored CGM profile for that previous meal. That stored CGM profile would include not only recorded glucose data for that meal, but would also include patient-specific insulin data values, such as the patient's basal rate, the patient's insulin sensitivity, and the patient's carb ratio. In accordance with a feature on the invention, the previously-stored similar profiles may be made available to the patient from the memory 611 on the display 608 or in printing, so that the patient may study them and select one, if desired. The patient may choose to assign those patient specific insulin values to determining the pre-meal insulin bolus for this new meal by selecting one of these stored profiles by a keyboard stroke, or by a visual touch panel stroke, or by other user interface means. Other exogenous patient data is also considered, such as insulin-on-board, in calculating the pre-meal bolus.

Stored profiles of previous events of this type are retrieved by the processor 728 from the memory 611 (FIG. 5). For example, the patient may retrieve all stored breakfast profiles. The processor then compares the stored profiles to one another to attempt to identify any persistent pattern. Such a pattern, as an example, may be seen in FIG. 2 in dashed line 232. Should the processor identify that in all stored profiles, or in a certain number of them, the patient's glucose level follows line 232 of FIG. 2, the processor may indicate that a persistent pattern exists in which the patient's glucose falls too low after consuming a meal. In this case, the processor would indicate in box 732 that a persistent pattern has been identified and would proceed to box 734 in which a change in the pre-meal insulin bolus is recommended so that a glucose level line more resembling 228 could be obtained. Such finding of a persistent pattern and recommended change in pre-meal insulin bolus may be presented to the patient by the display 608, it may also be stored in memory 611, it may be communicated to the patient's physician over a network 620 for storage and review by the physician or other health care provide, and/or may be printed for the patient.

The patient then has the option in this embodiment to accept the recommended exogenous data value change 736. If the change is accepted at box 736, such as a basal rate change, that change in the patient-specific insulin basal rate delivery will be implemented and the process begins again at box 720. However, if the comparison of multiple stored profiles 730 did not find a persistent pattern 738, no changes are recommended to the patient's exogenous insulin data and the process begins again at box 720.

While the disclosure has been particularly shown and described with reference to several embodiments thereof with particular details, it will be apparent to one of ordinary skill in the art that various changes may be made to these embodiments without departing from the principles of the invention, the scope of which is defined in the following claims .

## Claims

1. An integrated glucose monitoring system (600), comprising:
a memory (611) configured to store data relating to at least two glucose measurements of a user, a target glucose level (206, 226), and exogenous data, wherein the exogenous data comprises at least one of insulin on board data, meal data, prior carbohydrate intake data, or carbohydrate ratio data, wherein the at least two glucose measurements of the user are at different time points t1 and t2;
a user interface comprising a display (608);
a communication module configured to communicate with an insulin pump to acquire insulin delivery data; and
a processor (605) comprising computer-executable instructions to:
determine (701) a rate of change between the at least two glucose measurements of the user;
determine (702) a projected glucose level at a future time t3 based on at least the two glucose measurements, the determined rate of change, the exogenous data, and the acquired insulin delivery data; and
provide (707) a recommended therapeutic response if the projected glucose level at future time t3 exceeds the target glucose level.

2. The integrated glucose monitoring system of claim 1, wherein the insulin delivery data comprises the user's insulin-on-board data.

3. The integrated glucose monitoring system of claim 1 or 2, wherein t3 is up to 30 minutes after t2.

4. The integrated glucose monitoring system of any of claims 1-3, wherein the target glucose level is provided by a graphical user interface of the display.

5. The integrated glucose monitoring system of any of claims 1-4, wherein the processor provides the recommended therapeutic response if the projected glucose level at future time t3 exceeds the target glucose level by a preset value.

6. The integrated glucose monitoring system of claim 5, wherein the processor further comprises instructions configured to provide (705) an alarm if a difference between the projected glucose level and the target glucose level exceeds the preset value.

7. The integrated glucose monitoring system of any of claims 1-6, wherein the recommended therapeutic response comprises an insulin bolus or a temporary change to a basal rate.

8. The integrated glucose monitoring system of any of claims 1-7, wherein the at least two glucose measurements of the user are received from a continuous glucose monitoring sensor (601).

9. A method, comprising:
storing, in a memory (611), data relating to at least two glucose measurements of a user, a target glucose level (206, 226), and exogenous data, wherein the at least two glucose measurements of the user are at different time points t1 and t2, and wherein the exogenous data comprises at least one insulin on board data, meal data, prior carbohydrate intake data, or carbohydrate ratio data;
communicating with an insulin pump to acquire insulin delivery data;
determining (701), by a processor (605) coupled to the memory, a rate of change between the at least two glucose measurements of the user;
determining (702), by the processor, a projected glucose level at a future time t3 based on at least the two glucose measurements, the determined rate of change, the exogenous data, and the acquired insulin delivery data; and
providing (707) a recommended therapeutic response if the projected glucose level at future time t3 exceeds the target glucose level.

10. The method of claim 9, wherein the insulin delivery data comprises the user's insulin-on-board data.

11. The method of claim 9 or 10, wherein t3 is up to 30 minutes after t2.

12. The method of any of claims 9-11, wherein the recommended therapeutic response is provided if the projected glucose level at future time t3 exceeds the target glucose level by a preset value.

13. The method of claim 12, further comprising providing (705) an alarm if a difference between the projected glucose level and the target glucose level exceeds the preset value.

14. The method of any of claims 9-13, wherein the recommended therapeutic response comprises an insulin bolus or a temporary change to a basal rate.

15. A non-transitory computer-readable medium having instructions stored thereon that, when executed by at least one computing device (600), cause the at least one computing device to perform operations comprising:
store, in a memory (611) of the computer device, data relating to at least two glucose measurements of a user, a target glucose level (206, 226), and exogenous data, wherein the at least two glucose measurements of the user are at different time points t1 and t2, and wherein the exogenous data comprises at least one insulin on board data, meal data, prior carbohydrate intake data, or carbohydrate ratio data;
communicate with an insulin pump to acquire insulin delivery data;
determine (701) a rate of change between the at least two glucose measurements of the user;
determine (702) a projected glucose level at a future time t3 based on at least the two glucose measurements, the determined rate of change, the exogenous data, and the acquired insulin delivery data; and
provide (707) a recommended therapeutic response if the projected glucose level at future time t3 exceeds the target glucose level.

## Patentansprüche

1. Integriertes Glukoseüberwachungssystem (600), das Folgendes umfasst:
einen Speicher (611), konfiguriert zum Speichern von Daten in Bezug auf wenigstens zwei Glukosemessungen eines Benutzers, einen Zielglukosespiegel (206, 226) und exogene Daten, wobei die exogenen Daten mindestens eine von Daten zu mitgeführtem Insulin, Mahlzeitendaten, Daten zur vorherigen Kohlenhydrataufnahme oder Kohlenhydratverhältnisdaten umfassen, wobei die mindestens zwei Glukosemessungen des Benutzers zu unterschiedlichen Zeitpunkten t1 und t2 durchgeführt werden;
- eine Benutzeroberfläche, die eine Anzeige (608) umfasst;
ein Kommunikationsmodul, das konfiguriert ist zum Kommunizieren mit einer Insulinpumpe, um Daten zur Insulinabgabe zu erfassen; und
einen Prozessor (605), der computerausführbare Anweisungen umfasst zum:
Bestimmen (701) einer Änderungsrate zwischen den mindestens zwei Glukosemessungen des Benutzers;
Bestimmen (702) eines voraussichtlichen Glukosespiegels zu einem zukünftigen Zeitpunkt t3, basierend auf mindestens den beiden Glukosemessungen, der bestimmten Änderungsrate, den exogenen Daten und den erfassten Daten zur Insulinabgabe; und
Bereitstellen (707) einer empfohlenen therapeutischen Reaktion, wenn der voraussichtliche Glukosespiegel zum zukünftigen Zeitpunkt t3 den Zielglukosespiegel überschreitet.

2. Integriertes Glukoseüberwachungssystem nach Anspruch 1, wobei die Daten zur Insulinabgabe die Daten zu mitgeführtem Insulin des Benutzers umfassen.

3. Integriertes Glukoseüberwachungssystem nach Anspruch 1 oder 2, wobei t3 bis zu 30 Minuten nach t2 liegt.

4. Integriertes Glukoseüberwachungssystem nach einem der Ansprüche 1-3, wobei der Zielglukosespiegel über eine grafische Benutzeroberfläche der Anzeige bereitgestellt wird.

5. Integriertes Glukoseüberwachungssystem nach einem der Ansprüche 1-4, wobei der Prozessor die empfohlene therapeutische Reaktion bereitstellt, wenn der voraussichtliche Glukosespiegel zum zukünftigen Zeitpunkt t3 den Zielglukosespiegel um einen voreingestellten Wert überschreitet.

6. Integriertes Glukoseüberwachungssystem nach Anspruch 5, wobei der Prozessor ferner Anweisungen umfasst, konfiguriert zum Bereitstellen (705) eines Alarms, wenn eine Differenz zwischen dem voraussichtlichen Glukosespiegel und dem Zielglukosespiegel den voreingestellten Wert überschreitet.

7. Integriertes Glukoseüberwachungssystem nach einem der Ansprüche 1-6, wobei die empfohlene therapeutische Reaktion einen Insulinbolus oder eine vorübergehende Änderung einer Basalrate umfasst.

8. Integriertes Glukoseüberwachungssystem nach einem der Ansprüche 1-7, wobei die mindestens zwei Glukosemessungen des Benutzers von einem kontinuierlichen Glukoseüberwachungssensor (601) empfangen werden.

9. Verfahren, umfassend:
Speichern, in einem Speicher (611), von Daten in Bezug auf wenigstens zwei Glukosemessungen eines Benutzers, einen Zielglukosespiegel (206, 226) und exogene Daten, wobei die mindestens zwei Glukosemessungen des Benutzers zu unterschiedlichen Zeitpunkten t1 und t2 durchgeführt werden, und wobei die exogenen Daten mindestens eine von Daten zu mitgeführtem Insulin, Mahlzeitendaten, Daten zur vorherigen Kohlenhydrataufnahme oder Kohlenhydratverhältnisdaten umfassen;
Kommunizieren mit einer Insulinpumpe zum Erfassen von Daten zur Insulinabgabe;
Bestimmen (701), durch einen Prozessor (605), der mit dem Speicher gekoppelt ist, einer Änderungsrate zwischen den mindestens zwei Glukosemessungen des Benutzers;
Bestimmen (702), durch den Prozessor, eines voraussichtlichen Glukosespiegels zu einem zukünftigen Zeitpunkt t3, basierend auf mindestens den beiden Glukosemessungen, der bestimmten Änderungsrate, den exogenen Daten und den erfassten Daten zur Insulinabgabe; und
Bereitstellen (707) einer empfohlenen therapeutischen Reaktion, wenn der voraussichtliche Glukosespiegel zum zukünftigen Zeitpunkt t3 den Zielglukosespiegel überschreitet.

10. Verfahren nach Anspruch 9, wobei die Daten zur Insulinabgabe die Daten zu mitgeführtem Insulin des Benutzers umfassen.

11. Verfahren nach Anspruch 9 oder 10, wobei t3 bis zu 30 Minuten nach t2 liegt.

12. Verfahren nach einem der Ansprüche 9-11, wobei die empfohlene therapeutische Reaktion bereitgestellt wird, wenn der voraussichtliche Glukosespiegel zum zukünftigen Zeitpunkt t3 den Zielglukosespiegel um einen voreingestellten Wert überschreitet.

13. Verfahren nach Anspruch 12, ferner umfassend Bereitstellen (705) eines Alarms, wenn eine Differenz zwischen dem voraussichtlichen Glukosespiegel und dem Zielglukosespiegel den voreingestellten Wert überschreitet.

14. Verfahren nach einem der Ansprüche 9-13, wobei die empfohlene therapeutische Reaktion einen Insulinbolus oder eine vorübergehende Änderung einer Basalrate umfasst.

15. Nichtflüchtiges computerlesbares Speichermedium, das Anweisungen darauf gespeichert aufweist, die, wenn sie von mindestens einer Rechenvorrichtung (600) ausgeführt werden, die mindestens eine Rechenvorrichtung veranlassen, Operationen durchzuführen, umfassend:
Speichern, in einem Speicher (611) der Rechenvorrichtung, von Daten in Bezug auf wenigstens zwei Glukosemessungen eines Benutzers, einen Zielglukosespiegel (206, 226) und exogene Daten, wobei die mindestens zwei Glukosemessungen des Benutzers zu unterschiedlichen Zeitpunkten t1 und t2 durchgeführt werden, und wobei die exogenen Daten mindestens eine von Daten zu mitgeführtem Insulin, Mahlzeitendaten, Daten zur vorherigen Kohlenhydrataufnahme oder Kohlenhydratverhältnisdaten umfassen;
Kommunizieren mit einer Insulinpumpe zum Erfassen von Daten zur Insulinabgabe;
Bestimmen (701) einer Änderungsrate zwischen den mindestens zwei Glukosemessungen des Benutzers;
Bestimmen (702) eines voraussichtlichen Glukosespiegels zu einem zukünftigen Zeitpunkt t3, basierend auf mindestens den beiden Glukosemessungen, der bestimmten Änderungsrate, den exogenen Daten und den erfassten Daten zur Insulinabgabe; und
Bereitstellen (707) einer empfohlenen therapeutischen Reaktion, wenn der voraussichtliche Glukosespiegel zum zukünftigen Zeitpunkt t3 den Zielglukosespiegel überschreitet.

## Revendications

1. Système intégré de surveillance du glucose (600), comprenant :
une mémoire (611) configurée pour stocker des données correspondant à au moins deux mesures de glucose d'un utilisateur, un niveau de glucose cible (206, 226) et des données exogènes, dans lequel les données exogènes comprennent au moins une parmi des données d'insuline active, des données de repas, des données sur les apports préalables en glucides ou des données de ratio de glucides, dans lequel les au moins deux mesures de glucose de l'utilisateur sont à des moments différents t1 et t2 ;
- une interface utilisateur comprenant un affichage (608) ;
un module de communication configuré pour communiquer avec une pompe à insuline afin d'acquérir des données d'administration d'insuline ; et
un processeur (605) comprenant des instructions exécutables par ordinateur pour :
déterminer (701) une vitesse de changement entre les au moins deux mesures de glucose de l'utilisateur ;
déterminer (702) un niveau de glucose projeté à un moment futur t3 sur la base des au moins deux mesures de glucose, la vitesse de changement déterminée, les données exogènes et les données d'administration d'insuline acquises ; et
fournir (707) une réponse thérapeutique recommandée si le niveau de glucose projeté au moment futur t3 dépasse le niveau de glucose cible.

2. Système intégré de surveillance du glucose selon la revendication 1, dans lequel les données d'administration d'insuline comprennent les données d'insuline active de l'utilisateur.

3. Système intégré de surveillance du glucose selon la revendication 1 ou 2, dans lequel t3 est jusqu'à 30 minutes après t2.

4. Système intégré de surveillance du glucose selon l'une quelconque des revendications 1-3, dans lequel le niveau de glucose cible est fourni par une interface utilisateur graphique de l'affichage.

5. Système intégré de surveillance du glucose selon l'une quelconque des revendications 1-4, dans lequel le processeur fournit la réponse thérapeutique recommandée si le niveau de glucose projeté au moment futur t3 dépasse le niveau de glucose cible d'une valeur prédéfinie.

6. Système intégré de surveillance du glucose selon la revendication 5, dans lequel le processeur comprend en outre des instructions configurées pour fournir (705) une alarme si une différence entre le niveau de glucose projeté et le niveau de glucose cible dépasse la valeur prédéfinie.

7. Système intégré de surveillance du glucose selon l'une quelconque des revendications 1-6, dans lequel la réponse thérapeutique recommandée comprend un bolus d'insuline ou une modification provisoire d'un taux d'insuline basale.

8. Système intégré de surveillance du glucose selon l'une quelconque des revendications 1-7, dans lequel les au moins deux mesures de glucose de l'utilisateur sont reçues par un capteur de surveillance continue du glucose (601).

9. Procédé, comprenant :
le stockage dans une mémoire (611) de données correspondant à au moins deux mesures de glucose d'un utilisateur, à un niveau de glucose cible (206, 226) et à des données exogènes, dans lequel les au moins deux mesures de glucose de l'utilisateur sont à des moments différents t1 et t2, et dans lequel les données exogènes comprennent au moins une parmi des données d'insuline active, des données de repas, des données sur les apports préalables en glucides ou des données de ratio de glucides ;
la communication avec une pompe à insuline pour acquérir des données d'administration d'insuline ;
la détermination (701), par un processeur (605) couplé à la mémoire, d'une vitesse de changement entre au moins deux mesures de glucose de l'utilisateur ;
la détermination (702), par le processeur, d'un niveau de glucose projeté à un moment futur t3 sur la base des au moins deux mesures de glucose, la vitesse de changement déterminée, les données exogènes et les données d'administration d'insuline acquises ; et
la fourniture (707) d'une réponse thérapeutique recommandée si le niveau de glucose projeté au moment futur t3 dépasse le niveau de glucose cible.

10. Procédé selon la revendication 9, dans lequel les données d'administration d'insuline comprennent les données d'insuline active de l'utilisateur.

11. Procédé selon la revendication 9 ou 10, dans lequel t3 est jusqu'à 30 minutes après t2.

12. Procédé selon l'une quelconque des revendications 9-11, dans lequel la réponse thérapeutique recommandée est fournie si le niveau de glucose projeté au moment futur t3 dépasse le niveau de glucose cible d'une valeur prédéfinie.

13. Procédé selon la revendication 12, comprenant en outre la fourniture (705) d'une alarme si une différence entre le niveau de glucose projeté et le niveau de glucose cible dépasse la valeur prédéfinie.

14. Procédé selon l'une quelconque des revendications 9-13, dans lequel la réponse thérapeutique recommandée comprend un bolus d'insuline ou une modification provisoire d'un taux d'insuline basale.

15. Support non transitoire lisible par ordinateur présentant des instructions stockées sur celui-ci qui, lorsqu'elles sont exécutées par au moins un dispositif informatique (600), amènent le au moins un dispositif informatique à effectuer des opérations comprenant :
le stockage dans une mémoire (611) d'un dispositif informatique de données correspondant à au moins deux mesures de glucose d'un utilisateur, à un niveau de glucose cible (206, 226) et à des données exogènes, dans lequel les au moins deux mesures de glucose de l'utilisateur sont à des moments différents t1 et t2, et dans lequel les données exogènes comprennent au moins une parmi des données d'insuline active, des données de repas, des données sur les apports préalables en glucides ou des données de ratio de glucides ;
la communication avec une pompe à insuline pour acquérir des données d'administration d'insuline ;
la détermination (701) d'une vitesse de changement entre les au moins deux mesures de glucose de l'utilisateur ;
la détermination (702) d'un niveau de glucose projeté à un moment futur t3 sur la base des au moins deux mesures de glucose, la vitesse de changement déterminée, les données exogènes et les données d'administration d'insuline acquises ; et
la fourniture (707) d'une réponse thérapeutique recommandée si le niveau de glucose projeté au moment futur t3 dépasse le niveau de glucose cible.
